# EUROPEAN PATENT APPLICATION

(11) **EP 0 599 786 A1**
(43) Date of publication of application: **01.06.1994**
(21) Application number: 93830153.8
(22) Date of filing: 07.04.1993
(51) Int. Cl.: A61N 2/04, A61N 2/02

(54) **Electromagnetic dental mineralizer**

(30) Priority: 25.11.1992 IT CH920009
(71) Applicant: Diodato, Enzo Lino, I-66026 Ortona (CH) (IT)
(72) Inventor: Diodato, Enzo Lino, I-66026 Ortona (CH) (IT)

(57) **Abstract**

A mouth internal device is presented which generates a magnetic field in the oral cavity. Because of that an electrical field is induced with the result to stimulate in the tooth the accelerated growth of the dentine, with the aim of protecting the dental pulp in a series of pathological processes that can compromise its vitality.

## Description

It is known that a cell, in its most simple structure, can be represented as an aggregate of atoms and molecules; in other words, a typical electrochemical system dived into an electromagnetic field. It is evident thath modifying from outside the electrochemical environment in which the cell lives, its electrochemical bonds are changed, both at cellular membrane level and at more internal level, thus affecting cell's functions. This can be done putting the cell in an electromagnetic field produced by a field generator. Choosing an appropriate electromagnetic field (waveform, frequency, intensity, etc.) the cell is stimulated to modify its own natural functions during the application period. Of course the electromagnetic fields must be choosen in such a way that no damage will result for the cell.

Research in this field has already given interesting, even if not conclusive, biological and medical results indicating that cellular functions control is possible through electromagnetic fields especially in the reactivation of quiescent cells. The most relevant applications have occurred in orthopedia where healing times of fractured bones have drammatically improved with no collateral effects. As a matter of fact the electromagnetic fields makes the center of the fracture resume cellular activity, promoting calcium and other minerals flow inside the cell.

The field that accelerates these regenerative processes can be electrical or magnetic.

The electrical field is now almost no longer in use, because of a series of well known complications: the installation of the electrodes in the patient exposes him to the risks of an operation; the spontaneous rejection of the electrodes causes a reduction of the difference in potential between them; and, beside it, also the decalcification induced by the electrical currents must be considered. This is why it is so advantageous to induce electrical currents by means of magnetic fields.

The idea of the present patent is to use a very small device that can be introduced in the oral cavity in order to induce electrical currents in the teeth by means of a magnetic field.

Essentially two very small coils, fed by a programmable generator, create a magnetic field, perpendicular to tooth base.

This field determines, in the odontogenetic area, an acceleration of the dentine generation process thickening the area between the enamel (and anyway the external environment) and the pulp.

This regeneration process, probably, will proceed to a pace analogous or even faster than in orthopedia, because dentine is formed by elements in which calcium phosfate, water and calcium carbonate are present in larger percentage.

A dentine thickening process reduces the risks of permanent damage to the pulp, which may be caused by different types of pathological processes like caries, abrasions, fractures, demineralizations, and also by tooth stumping for anchorage to bridges and crowns. Of course with pulp protection the necessity of endodontic therapies, wich normally would mean an additional loss of good dental tissue, is strongly reduced.

Technical data which characterize the device will be presented at a later stage, even if, at this point it is well established that the generator frequency operates in the range of the cardiac pulse, in order to make the device action more affective.

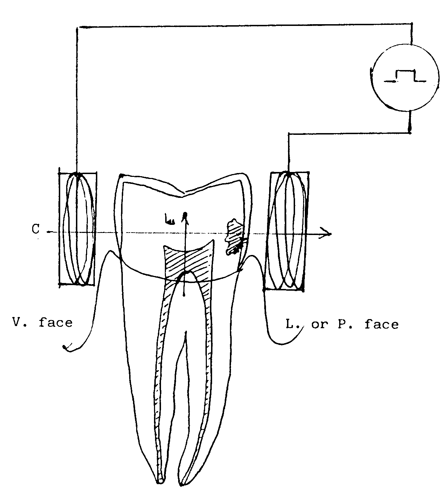

## Claims

**1)** The conception of activating/reactivating dentine growth stimulating odontogenetic areas with electromagnetic fields.

**2)** A mouth internal, battery operated, device which, with an impulse generator and two coils set on both tooth faces (the vestibular face and the palatine or lingual one), creates an electromagnetic field.
